# FASCICULE DE BREVET EUROPEEN

(11) **EP 1 086 044 B1**
(45) Date de publication et mention de la délivrance du brevet: **14.08.2002**
(21) Numéro de dépôt: 99917000.4
(22) Date de dépôt: 06.05.1999
(51) Int. Cl.: C01B 21/16

(54) **PROCEDE DE PREPARATION D'HYDRATE D'HYDRAZINE**
VERFAHREN ZUR HERSTELLUNG VON HYDRAZINHYDRAT
METHOD FOR PREPARING HYDRAZINE HYDRATE

(30) Priorité: 14.05.1998 FR 9806078
(43) Date de publication de la demande: 28.03.2001
(73) Titulaire: Atofina, 92800 Puteaux (FR)
(72) Inventeur: SCHIRMANN, Jean-Pierre, F-75011 Paris (FR)
(74) Mandataire: Dang, Doris
(86) Numéro de dépôt international: FR9901073
(87) Numéro de publication internationale: WO9958445

(56) Documents cités:
- EP-A- 0 294 100
- EP-A- 0 399 866
- US-A- 4 657 751
- US-A- 5 252 309
- DATABASE WPI Section Ch, Week 8128 Derwent Publications Ltd., London, GB; Class E36, AN 81-50501D XP002088233 & JP 56 059608 A (OTSUKA KAGAKU YAKUHIN KK), 23 mai 1981

## Description

La présente invention concerne un procédé de préparation d'hydrate d'hydrazine. La présente invention concerne plus précisément un procédé amélioré de fabrication d'hydrate d'hydrazine à partir d'azine de la méthyl éthyl cétone.

La production industrielle de l'hydrate d'hydrazine se fait selon les procédés RASCHIG, BAYER ou à l'eau oxygénée.

Dans le procédé RASCHIG, on oxyde l'ammoniac par un hypochlorite pour obtenir une solution diluée d'hydrate d'hydrazine qu'il faut ensuite concentrer par distillation. Ce procédé peu sélectif, peu productif et très polluant, n'est presque plus utilisé.

Le procédé BAYER est une variante du procédé RASCHIG qui consiste à déplacer un équilibre chimique en piégeant, à l'aide d'une cétone, l'hydrazine formée sous forme d'azine. L'azine est ensuite isolée puis hydrolysée en hydrate d'hydrazine. Les rendements sont améliorés, mais il n'y a pas d'amélioration quant aux rejets dans l'environnement.

Le procédé à l'eau oxygénée consiste à oxyder un mélange d'ammoniac et d'une cétone par l'eau oxygénée en présence d'un moyen d'activation de l'eau oxygénée pour faire directement l'azine qu'il suffit ensuite d'hydrolyser en hydrate d'hydrazine. Les rendements sont élevés et le procédé n'est pas polluant. Le procédé à l'eau oxygénée est utilisé par la demanderesse et est décrit dans de nombreux brevets, par exemple US 3 972 878, US 3 972 876, US 3 948 902 et US 4 093 656.

L'hydrolyse d'une azine en hydrate d'hydrazine est décrite dans les brevets US 4 724 133 SCHIRMANN et al, US 4 725 421 SCHIRMANN et al et GB 1 164 460. Cette hydrolyse s'effectue dans une colonne de distillation qu'on alimente avec de l'eau et de l'azine, en tête on récupère la cétone et en pied l'hydrate d'hydrazine.

EP 70 155 décrit aussi un autre procédé à l'eau oxygénée.

Ces procédés sont aussi décrits dans ULLMANN'S ENCYCLOPEDIA OF INDUSTRIAL CHEMISTRY (1989), vol A 13, pages 182-183 et les références incluses.

Dans les procédés à l'eau oxygénée, on oxyde l'ammoniac par le peroxyde d'hydrogène en présence d'une cétone et d'un moyen d'activation du peroxyde d'hydrogène selon la réaction globale suivante, en faisant une azine :

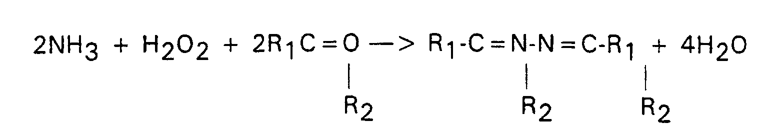

Le moyen d'activation peut être un nitrile, un amide, un acide carboxylique ou encore un dérivé du sélénium, de l'antimoine ou de l'arsenic. Puis l'azine est hydrolysée en hydrazine et la cétone régénérée est recyclée selon la réaction suivante :

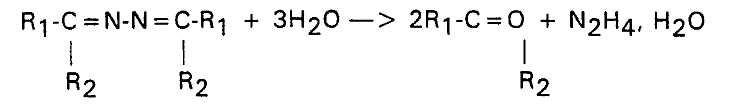

Cette hydrolyse s'effectue dans une colonne à distiller, on récupère la cétone en tête et l'hydrate d'hydrazine en pied.

Que l'azine provienne d'un procédé à l'eau oxygénée ou d'un autre mode de préparation, il est nécessaire d'effectuer l'hydrolyse de cette azine pour obtenir l'hydrate d'hydrazine. La présente invention concerne l'hydrolyse de la méthyl éthyl cétone azine (aussi appelée mécazine dans la suite du texte), quelle que soit sa provenance pour la transformer en hydrate d'hydrazine. La demanderesse a découvert que, pendant l'hydrolyse, il se formait des hétérocycles de la famille de la pyrazoline et que, si on ne purge pas les produits, l'hydrate d'hydrazine est coloré.

La présente invention concerne un procédé de préparation d'hydrate d'hydrazine dans lequel on hydrolyse de la méthyl éthyl cétone azine pour obtenir de l'hydrate d'hydrazine et de la méthyl éthyl cétone, caractérisé en ce qu'on purge des hétérocycles de la famille de la pyrazoline en quantité suffisante pour éviter la coloration de l'hydrate d'hydrazine.

Que l'azine soit produite par un procédé à l'eau oxygénée ou un autre procédé, on utilise avantageusement la méthyl éthyl cétone parce qu'elle est peu soluble dans le milieu aqueux.

Dans le procédé à l'eau oxygénée, l'azine de la méthyl éthyl cétone a été choisie industriellement parce qu'elle est relativement insoluble dans le milieu réactionnel qui est forcément aqueux puisque l'on utilise des solutions aqueuses commerciales de peroxyde d'hydrogène titrant entre 30 et 70 % en poids. Cette azine est donc facilement récupérable et séparable par simple décantation. Elle est très stable surtout en milieu alcalin, c'est-à-dire dans le milieu réactionnel ammoniacal. Dans tous les procédés modernes, cette azine est ensuite purifiée, puis hydrolysée dans une colonne de distillation réactive pour libérer "in fine" de la méthyl éthyl cétone en tête à recycler à la réaction de fabrication, et surtout une solution aqueuse de l'hydrate d'hydrazine en pied, qui doit contenir aussi peu de produits carbonés que possible comme impuretés et doit être incolore.

Bien que des colonnes classiques à garnissage puissent convenir, on utilise en général des colonnes à plateaux. Suivant le temps de séjour admis sur les plateaux et la pression, donc les températures auxquelles on opère, le nombre de plateaux peut varier énormément. Dans la pratique, lorsqu'on opère sous une pression de 8 à 10 bars, le nombre de plateaux nécessaires est de l'ordre de 40 à 50.

Cette colonne est opérée en continu et l'on injecte les réactifs c'est-à-dire la mécazine et l'eau dans la partie haute de la colonne.

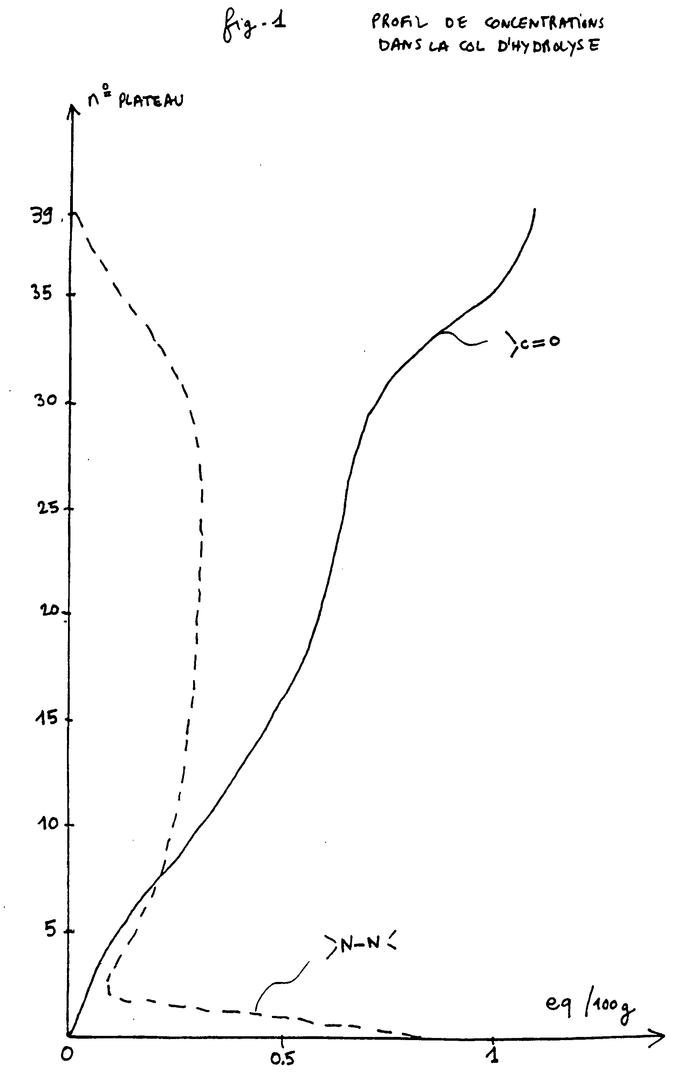

Or, la demanderesse a découvert que, lorsqu'on pratique l'hydrolyse de la mécazine selon les réactions équilibrées suivantes : 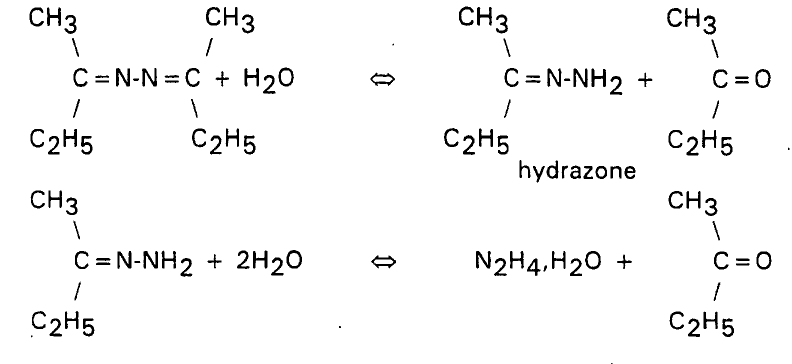 on parvient bien à ses fins comme l'indique la figure 1 qui représente les variations en groupements 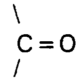 et en groupements N-N tout au long de la colonne. La quantité des groupements est exprimée en équivalent molaire pour 100 g de l'échantillon considéré. Les groupements carbonyles 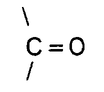 sont dosés par oximation, alors que les groupements N-N sont dosés par iodométrie classique après hydrolyse par l'acide sulfurique selon les techniques connues de l'homme de l'art. Le plateau 1 est en bas de la colonne, le plateau 40 est en tête. On constate bien que l'hydrolyse est totale puisque la teneur en groupements 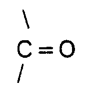 dans le pied est nulle, de même que la teneur en groupements N-N en tête est également nulle.

Cependant, la demanderesse a aussi constaté que, si l'on n'y prenait garde, bien que l'hydrolyse de la mécazine soit complète, on obtient en pied une solution d'hydrate d'hydrazine non pas incolore, mais colorée et parfois fortement, et cela en raison d'une teneur en produits carbonés parfois fort importante. Contrairement à ce que l'on pourrait penser, ces produits carbonés ne sont ni de l'azine de la méthyl éthyl cétone ni de l'hydrazone, mais des hétérocycles dérivés de la mécazine : la triméthyl -3,4,5 éthyl-5 pyrazoline 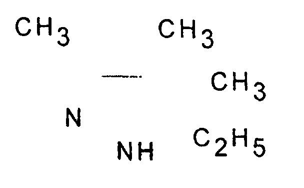 et la diéthyl -3,5 méthyl 5 pyrazoline 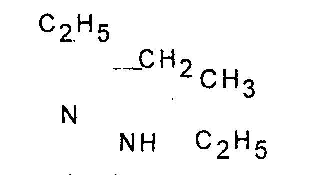 Ces composés, isomères de la mécazine, se forment dans la colonne d'hydrolyse, et sont responsables des colorations jaunes, voire brunes ou rouges que l'on peut observer.

Cette hydrolyse s'effectue par exemple dans une colonne à plateaux ou à garnissage de type colonne de distillation qu'on alimente avec l'azine et de l'eau, on obtient (i) en tête, la méthyl éthyl cétone avec de l'eau puisqu'il se forme un azéotrope avec l'eau et (ii) en pied, une solution aqueuse d'hydrate d'hydrazine.

L'hydrolyse des azines est connue. Par exemple, E.C. GILBERT, dans un article dans le Journal of American Chemical Society vol.51, pages 3397-3409(1929), décrit les réactions équilibrées de formation d'azine et les réactions d'hydrolyse de celles-ci. L'hydrolyse doit être réalisée dans une colonne réactive, de telle sorte qu'en séparant continuellement la méthyl éthyl cétone en tête de colonne de distillation et l'hydrate d'hydrazine en pied de colonne, on peut parvenir à une hydrolyse totale. Bien entendu ce système est au meilleur de son fonctionnement lorsqu'on travaille en continu comme décrit dans les brevets français 1 315 348 ou anglais 1 211 547, ou encore le brevet US 4 725 421.

Dans tous ces brevets, la réaction est réalisée dans une colonne de distillation à garnissage ou mieux à plateaux fonctionnant sous une pression de 2 à 25 bars et avec une température de pied de 150°C à 200°C.

Dans cette colonne, s'effectue l'hydrolyse de l'azine et la séparation de l'hydrate d'hydrazine d'avec la méthyl éthyl cétone. Ces conditions sont connues. L'homme de métier détermine facilement le nombre de plateaux ou la hauteur de garnissage, ainsi que les points d'alimentation en azine et en eau. On obtient en pied des solutions à 30 ou même jusqu'à 45% en poids d'hydrate d'hydrazine. Ce rapport molaire eau/azine à l'alimentation de cette colonne est au moins supérieur à la stoechiométrie et avantageusement compris entre 5 et 7,75. Le pied de colonne est entre 150°C et 200°C, de préférence 175 à 190°C. La pression est fonction de la température d'ébullition de l'azi de l'eau et de la méthyl éthyl cétone. Une telle hydrolyse est décrite dans US 4 725 721.

Selon le nombre de plateaux ou la hauteur de garnissage, la position de l'alimentation en azine et la position de l'alimentation en eau, le reflux, la nature de l'azine etc..., l'homme du métier peut déterminer facilement dans quelle partie de la colonne on obtient la concentration maximum en triméthyl-3,4,5 éthyl-5 pyrazoline et diéthyl-3,5 méthyl 5 pyrazoline.

La demanderesse vient de découvrir aussi que si cette formation parasite ne peut être anihilée, on peut du moins éviter que ces produits se retrouvent dans le pied de la colonne, en opérant un soutirage latéral au niveau de quelques plateaux. Avantageusement, on effectue des soutirages sur les plateaux où la concentration en hétérocycles de la famille de la pyrazoline est maximum. Le soutirage peut être fait de façon permanente, mais on préfère opérer de façon non continue, de façon à éviter de perdre d'autres produits au processus réactionnel.

On soutire des quantités du milieu présent dans la colonne d'hydrolyse de telle sorte que la concentration moyenne de ces hétérocycles dans le ventre de concentration ne dépasse pas 4 % et de préférence 2 % (en poids). La quantité à soutirer se détermine facilement par analyse par chromatographie en phase gazeuse de la concentration de ces hétérocycles. Ces valeurs sont suffisantes pour éviter de colorer l'hydrate d'hydrazine produit en pied.

La présente invention concerne aussi un procédé de préparation d'hydrate d'hydrazine dans lequel :
(a) on fait réagir l'ammoniac, le peroxyde d'hydrogène et la méthyl éthyl cétone (MEK) en présence d'une solution de travail pour former une azine ;
(b) on sépare la solution de travail et l'azine contenant éventuellement de la méthyl éthyl cétone n'ayant pas réagi ;
(c) on recycle la solution de travail à l'étape (a) après un traitement éventuel ;
(d) on hydrolyse l'azine pour obtenir de l'hydrate d'hydrazine et régénérer la méthyl éthyl cétone ;
(e) on recycle à l'étape (a) la méthyl éthyl cétone, ce procédé étant caractérisé en ce que, dans l'étap (d), on effectue une purge d'hétérocycles de la famille de la pyrazoline.

### Etape (a)

L'eau oxygénée peut être utilisée sous la forme commerciale habituelle, par exemple en solution aqueuse contenant entre 30 et 90 % en poids de H₂O₂. Avantageusement on peut ajouter un ou plusieurs stabilisants usuels des solutions peroxydiques, par exemple de l'acide phosphorique, pyrophosphorique, citrique, nitrilo-triacétique, éthylènediaminetétracétique ou les sels d'ammonium ou de métal alcalin de ces acides. La quantité à utiliser est avantageusement comprise entre 10 et 1000 ppm et, de préférence, entre 50 et 250 ppm de l'ensemble des réactifs et de la solution de travail à l'entrée du réacteur. L'ammoniac peut être anhydre ou en solution aqueuse.

La solution de travail contient un moyen d'activation de l'eau oxygénée, c'est-à-dire un produit tel qu'on puisse produire de l'azine à partir d'ammoniac, d'eau oxygénée et de méthyl éthyl cétone.

Cet activateur peut être choisi parmi les oxyacides organiques ou inorganiques, leurs sels d'ammonium et d'une façon générale leurs dérivés : anhydrides, esters, amides, nitriles, peroxydes d'acyle, ou leurs mélanges. Avantageusement, on utilise les amides, les sels d'ammonium et les nitriles.

À titre d'exemple, on peut citer (i) des amides d'acides carboxyliques de formule R₅COOH dans laquelle R₅ est l'hydrogène, un radical alkyle linéaire ayant de 1 à 20 atomes de carbone, ou un radical alkyle ramifié ou cyclique ayant de 3 à 12 atomes de carbone, ou un radical phényle pouvant être substitué, (ii) des amides d'acides polycarboxyliques de formule R₆(COOH)ₙ dans laquelle R₆ représente un radical alkylène ayant de 1 à 10 atomes de carbone et n étant un nombre entier supérieur ou égal à 2 ; R₆ peut être une simple liaison, alors n vaut 2. Les radicaux R₅ et R₆ peuvent être substitués par des halogènes, des groupements OH, NO₂ ou méthoxy. On peut citer aussi les amides des acides organiques de l'arsenic. Les acides organiques de l'arsenic sont, par exemple, l'acide méthylarsonic, l'acide phénylarsonic et l'acide cacodylique.

Les amides préférés sont le formamide, l'acétamide, le monochloracétamide et le propionamide.

Parmi les sels d'ammonium, on utilise avantageusement les sels d'hydracides, d'oxyacides minéraux, d'acides arylsulfoniques, d'acides R₅COOH ou d'acides R₆(COOH)ₙ, R₅, R₆ et n étant définis précédemment, des acides organiques de l'arsenic.

Les sels d'ammonium préférés sont le formiate, l'acétate, le monochloracétate, le propionate, le phénylarsonate et le cacodylate. Parmi les nitriles, on peut citer avantageusement les produits de formule R₇(CN)ₙ, n pouvant varier de 1 à 5 selon la valence de R₇, R₇ étant un alkyle cyclique ou non cyclique ayant de 1 à 12 atomes de carbone ou de benzène ou de la pyridine. R₇ peut être substitué par des groupements qui ne s'oxydent pas dans le réacteur de l'étape a, par exemple des halogènes, des groupes carboxyliques, esters carboxyliques, nitro, amine, hydroxy ou acide sulfonique.

Les nitriles préférés sont l'acétonitrile et le propionitrile.

On forme la solution de travail en mettant en solution un ou plusieurs produits choisis parmi les oxyacides organiques ou inorganiques, leurs sels d'ammonium et d'une façon générale leurs dérivés : anhydrides, esters, amides, nitriles, peroxydes d'acyle, ou leurs mélanges. Avantageusement, on utilise les amides, les sels d'ammonium ou les nitriles précédents.

Cette solution peut être aqueuse ou à base d'un alcool ou d'un mélange d'alcool et eau. Parmi les alcools, on utilise avantageusement les alcools aliphatiques saturés ayant de 1 à 6 atomes de carbone et, de préférence, 1 à 2 atomes de carbone.

On utilise aussi avantageusement des diols et plus particulièrement des diols ayant de 2 à 5 atomes de carbone. On peut citer par exemple le glycol, le propylèneglycol, le 1,3-propanediol, les 1,3- et 1,4- butanediols et le 1,5 pentanediol.

Selon une forme avantageuse de l'invention, la solution de travail est une solution alcoolique d'un acide organique de l'arsenic et est décrite dans le brevet EP 70 155 dont le contenu est incorporé dans la présente demande. Selon une autre forme avantageuse de l'invention, la solution de travail est une solution aqueuse d'un amide d'acide faible et du sel d'ammonium correspondant à cet acide, telle que décrite dans le brevet EP 487 160.

Ces amides d'acides faibles sont dérivés des acides carboxyliques correspondants qui ont une constante de dissociation inférieure à 5 x 10⁻⁵, c'est-à-dire les acides qui ont un pK plus grand que 4,3 en solution aqueuse à 25°C.

Pour les acides polycarboxyliques, ce sont les acides dont la constante de la première ionisation est inférieure à 5 x 10⁻⁵.

À titre d'exemple, on peut citer les acides carboxyliques de formule R₈COOH dans laquelle R₈ est un radical alkyle linéaire ayant de 1 à 20 atomes de carbone, ou un radical alkyle ramifié ou cyclique ayant de 3 à 12 atomes de carbone, ou un radical phényle pouvant être substitué, des acides polycarboxyliques de formule R₉(COOH)ₙ dans laquelle R₉ représente un radical alkylène ayant de 1 à 10 atomes de carbone et n étant un nombre entier supérieur ou égal à 2 ; R₉ peut être une simple liaison alors n vaut 2. Les radicaux R₈ et R₉ peuvent être substitués par des halogènes, des groupements OH, NO₂ ou méthoxy. On utilise de préférence l'acétamide, le propionamide, le n-butyramide ou l'isobutyramide.

Le sel d'ammonium correspondant de l'acétamide est l'acétate d'ammonium.

On ne sortirait pas du cadre de l'invention en formant le sel d'ammonium in situ c'est-à-dire en utilisant l'acide carboxylique correspondant qui donne par réaction avec l'ammoniac le sel d'ammonium.

Les proportions de l'amide et du sel d'ammonium correspondant peuvent varier dans de larges limites. On utilise habituellement de 1 à 25 parties du sel d'ammoniumpour 5 parties d'amide et de préférence 2 à 10.

Les réactifs peuvent être utilisés en quantités stoechiométriques, cependant on utilise par mole d'eau oxygénée 0,2 à 5 moles et de préférence 1,5 à 4 moles de méthyl éthyl cétone ; 0,1 à 10 moles et de préférence 1,5 à 4 moles d'ammoniac. La quantité de solution de travail est comprise entre 0,1 et 1 kg par mole d'eau oxygénée. Cette quantité dépend de sa qualité, c'est-à-dire de sa force catalytique ou son activité qui permet de convertir les réactifs en azine. Les proportions des réactifs fixées ci-dessus permettent d'obtenir une conversion totale de l'eau oxygénée et une production d'azine correspondant à plus de 50% de l'eau oxygénée engagée et pouvant atteindre 90 %.

La mise en contact de l'eau oxygénée, de l'ammoniac, de la MEK avec la solution de travail peut s'effectuer de façon quelconque.

Avantageusement, on opère dans un milieu homogène ou dans un milieu qui assure au moins une solubilisation suffisante des réactifs pour pouvoir obtenir de l'azine. La réaction peut se faire dans une très large plage de température, par exemple entre 0 et 100°C, et on opère avantageusement entre 30 et 70°C. Bien qu'on puisse opérer à toute pression, il est plus simple d'être à la pression atmosphérique, mais on peut monter jusqu'à environ 10 bars si c'est nécessaire pour maintenir de préférence la réaction de l'étape a en phase liquide.

Les réactifs peuvent être introduits simultanément ou séparément et dans un ordre quelconque dans la solution de travail. On peut utiliser toutes sortes de réacteurs, agités ou non agités, ou même de simples capacités qu'on peut disposer en parallèle, en série, à co-courant ou à contre-courant, ou tout combinaison de ces possibilités.

### Etape (b)

On sépare (i) l'azine et éventuellement l'excès de MEK et (ii) la solution de travail par des moyens connus tels que l'extraction liquide-liquide, la distillation, la décantation ou toute combinaison de ces possibilités.

Dans l'étape (c), la solution de travail peut être traitée.

Les étapes (a), (b) et (c) sont décrites par exemple dans les brevets EP 399 866 et EP 518 728 dont le contenu est incorporé dans la présente demande.

L'étape (d) a été décrite plus haut.

### Exemples

Dans une colonne en acier inoxydable 316 L de hauteur 3 m, de diamètre Ø = 70 mm équipée de 40 plateaux à monocloche perforée de diamètre 27 mm et espacés chacun de 80 mm. Les trous de chaque cloche ont un diamètre de 2 mm et sont au nombre de 14. Le volume utile de liquide retenu sur le plateau est de 33 ml. Il peut être ajusté en jouant sur la hauteur du déversoir.

Cette colonne est équipée de prises de températures (thermocouples) sur les plateaux 3, 6, 10, 13, 15, 19, 26, 28, 31 et 37 ainsi qu'en tête et en pied. Des sorties de soutirage équipées de vannes sont installées aux plateaux 3, 10, 19, 26, 31 et 37.

L'introduction des réactifs est possible au niveau des plateaux 5, 8, 12, 19, 22, 26 et 34. Le débit de reflux est mesuré à l'aide d'un rotamètre préalablement étalonné. L'apport de calories en pied de colonne est assuré par un chauffage électrique. L'adiabaticité du fût de la colonne est assurée par une gaine d'air chaud obtenue par chauffage électrique de façon à équilibrer les températures à l'intérieur et à l'extérieur de la colonne.

L'alimentation des réactifs est réalisée par pompe doseuse.

Le condenseur est alimenté par une circulation d'huile chaude maintenue entre 130 et 140°C.

Le mode opératoire retenu est le suivant :

On place dans le bouilleur de volume 800 cm³ de la colonne 400 cm³ d'eau bipermutée. L'ensemble de l'installation étant fermé, on démarre le chauffage et on laisse monter la pression jusqu'à 8 bars. Puis au fur et à mesure que l'eau monte dans la colonne on purge les inertes en maintenant la pression à 8 bars. Lorsque le niveau du bouilleur atteint 200 cm³, on commence l'injection d'eau au 34ème plateau pour continuer la constitution du ballast d'eau dans la colonne à raison de 645 g en 1 h 15 minutes. Lorsque la température atteint 162°C au 19ème plateau, on commence l'injection d'azine au 26ème plateau à raison de 543 g de solution d'azine à 82,4 % (3,2 moles) en 1 h 30 minutes. On continue à purger les inertes de la colonne en maintenant la pression à 8 bars. On laisse s'installer le reflux et on laisse à reflux total jusqu'à ce que le liquide de reflux soit homogène. Puis on démarre l'introduction continue des réactifs et le soutirage en pied et en tête. On opère à reflux 1. L'azine est introduite sous forme de mélange avec de la MEK à raison de 275,4 g/h (titrant 82,4 % en azine) et de 289 g/h d'eau bipermutée.

Compte tenu de ce qui a été dit précédemment sur les conditions de formation de la pyrazoline dans la colonne, il est absolument nécessaire d'éviter à tout prix la retombée d'azine vers le pied. La procédure d'arrêt utilisée consiste à :
- stopper l'introduction d'azine
- poursuivre l'introduction d'eau
- poursuivre la distillation et l'élimination de MEK jusqu'à ce que la température en tête atteigne 170°C, point d'ébullition de l'eau sous 8 bars.

La température de tête s'établit à 148°C alors que la température de pied est de 180 - 181 °C. En régime stationnaire on soutire du pied 200 g/h d'une solution d'hydrate d'hydrazine incolore à 35 %. La teneur en pyrazolines au plateau 19 est de 2,6 % et l'on effectue sur ce plateau un soutirage de 20 ml toutes les 4 heures d'un mélange d'azine de la MEK, de pyrazolines et d'eau.

En tête on soutire l'azéotrope MEK - eau, ce qui représente après refroidissement une phase organique de 300 g/h titrant 86,5 % en MEK et une phase aqueuse de 55 g/h titrant 26 % en MEK.

## Revendications

1. Procédé de préparation d'hydrate d'hydrazine dans lequel on hydrolyse de la méthyl éthyl cétone azine dans une colonne pour obtenir de l'hydrate d'hydrazine en pied et de la méthyl éthyl cétone en tête, **caractérisé en ce qu'**on purge des hétérocycles de la famille de la pyrazoline, en quantité suffisante pour éviter la coloration de l'hydrate d'hydrazine, en soutirant latéralement lesdits hétérocycles de la colonne d'hydrolyse.

2. Procédé de préparation d'hydrate d'hydrazine dans lequel:
(a) on fait réagir l'ammoniac, le peroxyde d'hydrogène et la méthyl éthyl cétone (MEK) en présence dune solution de travail pour former une azine ;
(b) on sépare la solution de travail d'avec l'azine contenant éventuellement la méthyl éthyl cétone n'ayant pas réagi ;
(c) on recycle la solution de travail à l'étape (a) après un traitement éventuel ;
(d) on hydrolyse l'azine dans une colonne pour obtenir de l'hydrate d'hydrazine en pied et régénérer la méthyl éthyl cétone en tête,
(e) on recycle à l'étape (a) la méthyl éthyl cétone, ce procédé étant **caractérisé en ce que**, dans l'étape (d), on effectue une purge d'hétérocycles de la famille de la pyrazoline, en soutirant latéralement lesdits hétérocycles de la colonne d'hydrolyse.

3. Procédé selon Tune quelconque des revendications précédentes dans lequel les hétérocycles de la famille de la pyrazoline sont la triméthyl-3,4,5 éthyl-5 pyrazoline et la diéthyl-3,5 méthyl-5 pyrazoline.

## Claims

1. Process for the preparation of hydrazine hydrate in which methyl ethyl ketone azine is hydrolysed in a column in order to obtain hydrazine hydrate at the bottom and methyl ethyl ketone at the top, **characterized in that** heterocycles of the pyrazoline family are bled off in an amount sufficient to prevent coloration of the hydrazine hydrate by taking a sidestream of the said heterocycles from the hydrolysis column .

2. Process for the preparation of hydrazine hydrate, in which:
(a) ammonia, hydrogen peroxide and methyl ethyl ketone (MEK) are reacted in the presence of a working solution in order to form an azine;
(b) the working solution is separated from the azine, which optionally comprises unreacted methyl ethyl ketone;
(c) the working solution is recycled to the stage (a) after an optional treatment;
(d) the azine is hydrolysed in a column in order to obtain hydrazine hydrate at the bottom and to regenerate the methyl ethyl ketone at the top,
(e) the methyl ethyl ketone is recycled to the stage (a), this process being **characterized in that**, in the stage (d), heterocycles of the pyrazoline family are bled off by taking a sidestream of the said heterocycles from the hydrolysis column.

3. Process according to either one of the preceding claims, in which the heterocycles of the pyrazoline family are 3,4,5-trimethyl-5-ethyl-pyrazoline and 3,5-diethyl-5-methylpyrazoline.

## Patentansprüche

1. Verfahren zur Herstellung von Hydrazinhydrat, bei dem Methylethylketonazin in einer Kolonne hydrolysiert wird, wodurch am Fuß Hydrazinhydrat und am Kopf Methylethylketon erhalten wird, **dadurch gekennzeichnet, daß** Heterocyclen aus der Pyrazolin-Familie in einer ausreichenden Menge entfernt werden, um die Färbung des Hydrazinhydrats zu verhindern, indem die Heterocyclen seitlich aus der Hydrolyse-Kolonne abgezogen werden.

2. Verfahren zur Herstellung von Hydrazinhydrat, bei dem
(a) Ammoniak, Wasserstoffperoxid und Methylethylketon (MEK) in Gegenwart einer Arbeitslösung umgesetzt werden, um ein Azin zu erzeugen;
(b) die Arbeitslösung von dem Azin getrennt wird, das gegebenenfalls das Methylethylketon enthält, das nicht abreagiert hat,
(c) die Arbeitslösung nach einer möglichen Behandlung in den Schritt (a) zurückgeführt wird,
(d) das Azin in einer Kolonne hydrolysiert wird unter Erhalt des Hydrazinhydrats am Fuß der Kolonne und unter Rückgewinnung des Methylethylketons am Kopf der Kolonne,
(e) das Methylethylketon in den Schritt (a) zurückgeführt wird,
wobei das Verfahren **dadurch gekennzeichnet ist, daß** in Schritt (d) Heterocyclen aus der Pyrazolin-Familie entfernt werden, indem die Heteroyclen seitlich aus der Hydrolyse-Kolonne abgezogen werden.

3. Verfahren nach einem der vorhergehenden Ansprüche, wobei es sich bei den Heterocyclen aus der Pyrazolin-Familie um 3,4,5-Trimethyl-5-ethylpyrazolin und 3,5-Diethyl-5-methylpyrazolin handelt.
